# EUROPEAN PATENT SPECIFICATION

(11) **EP 4 274 821 B1**
(45) Date of publication and mention of the grant of the patent: **05.03.2025**
(21) Application number: 22700471.0
(22) Date of filing: 06.01.2022
(51) Int. Cl.: C07C 51/41, C07C 55/07

(54) **PROCESS FOR PREPARING OXALATE AND OXALIC ACID WITH HYDRIDE CATALYST**
VERFAHREN ZUR HERSTELLUNG VON OXALAT UND OXALSÄURE MIT HYDRIDKATALYSATOR
PROCÉDÉ DE PRÉPARATION D'OXALATE ET D'ACIDE OXALIQUE AVEC CATALYSEUR HYBRIDE

(30) Priority: 07.01.2021 EP 21150580
(43) Date of publication of application: 15.11.2023
(73) Proprietor: Avantium Knowledge Centre B.V., 1014 BV Amsterdam (NL)
(72) Inventor: GRUTER, Gerardus Johannes Maria, 1014 BV Amsterdam (NL); SCHULER, Eric, 1012 WX Amsterdam (NL)
(74) Representative: Avantium Intellectual Property
(86) International application number: PCT/EP2022/050181
(87) International publication number: WO 2022/148797

(56) References cited:
- WO-A1-2016/124646
- LAKKARAJU ET AL.: "Formate to Oxalate: A Crucial Step for the Conversion of Carbon Dioxide into Multi-carbon Compounds", CHEMCATCHEM, vol. 8, 2016, pages 3453 - 3457, XP002803419

## Description

### FIELD OF THE INVENTION

The present invention relates to processes for preparing potassium oxalate and oxalic acid.

### BACKGROUND TO THE INVENTION

The use of fossil fuels in the production of chemicals for use per se or for further chemical conversion produces carbon dioxide which nowadays is considered undesirable. Therefore, substantial research is being carried out to convert renewable resources such as carbon dioxide into compounds conventionally made from fossil fuel. This is preferably done with the help of energy also supplied from renewable sources. It remains an outstanding challenge to generate from such renewable resources compounds containing multiple carbon atoms such as oxalate and oxalic acid. One possible route to oxalate is the coupling of two formate molecules.

While overall formate coupling provides an interesting pathway to oxalate, a drawback is still the high temperature and the relatively long reaction times needed for the formate to oxalate conversion. In addition, undesired carbonate formation is still often a problem in formate coupling. This will require a downstream purification step.

Lakkaraju et al. in ChemCatChem 2016, 3453-3457 describe catalytic conversion of molten sodium formate into oxalate salts. It is reported that strong base such as NaH or NaOH convert formate into oxalate at high temperatures such as 390°C. At temperatures below 350 °C, the conversion is described to be negligible.
L.K. Freidlin et al. published several papers on formate coupling. The effect of various caustic catalysts is described in L.K. Freidlin, Zhurnal Prikl. Khimii 1938, 11, 975-980.

It would be advantageous to have a process which would allow preparing oxalate at reduced temperature. It would be advantageous if oxalate could be prepared at reduced catalyst concentration. It furthermore would be advantageous if a higher yield could be obtained and/or the potassium formate coupling can be improved in any other way.

### SUMMARY OF THE INVENTION

Accordingly, the present invention provides a process for preparing potassium oxalate which process comprises contacting potassium formate with a base catalyst at a temperature of from 140 to 260°C which base catalyst is a metal hydride.

Furthermore, a process is provided for preparing oxalic acid which process comprises (i) preparing potassium oxalate in a process according to the invention, and (ii) converting the potassium oxalate to oxalic acid. Such conversion preferably is carried out by an electrochemical process.

### DETAILED DESCRIPTION OF THE INVENTION

Potassium formate for use in the process can be obtained from any source known to the person skilled in the art. An advantageous source would be producing metal formate from a carbon source such as formic acid, formaldehyde, CO, polyol and/or sugar.

Potassium formate can be prepared by reacting carbon monoxide with potassium hydroxide. An alternative method for preparing potassium formate is reacting carbon monoxide with potassium carbonate. A further method for preparing potassium formate is reacting carbon monoxide with potassium bicarbonate, also referred to as potassium hydrogen carbonate. This reaction may or may not require a catalyst. Formate also can be prepared by reacting gaseous formaldehyde with potassium hydroxide.

It is contemplated to use potassium formate prepared in an electrochemical process such as potassium formate obtained by a process involving electrochemical reduction of carbon dioxide. A preferred method for preparing potassium formate is described in WO2017/121887. This process comprises converting potassium carbonate and/or potassium bicarbonate to potassium formate. The carbonate and/or bicarbonate can have been prepared from so-called synthesis gas comprising carbon monoxide and hydrogen.

The base catalyst for use in the process is a metal hydride. Preferably, the base catalyst is selected from the group consisting of alkali metal hydrides. The alkali metal hydride preferably is selected from the group consisting of lithium hydride, sodium hydride and potassium hydride. The base catalyst preferably is sodium hydride.

The amount of base catalyst preferably is at most 5 % by weight based on total amount of potassium formate, more preferably at most 4 % by weight, more preferably at most 3 % by weight, more preferably at most 2.5 % by weight, more preferably at most 2 % by weight and more preferably at most 1 % by weight. The amount of base catalyst preferably is at least 0.01 % by weight, more preferably at least 0.02 % by weight, more preferably at least 0.05 % by weight, more preferably at least 0.1 % by weight and more preferably at least 0.2 % by weight.

The mixture containing base catalyst and potassium formate preferably is substantially liquid during reaction. The presence of further compounds can change the temperature at which the reaction mixture is in the liquid phase. If no further compounds are present, the process preferably is carried out at a temperature above the melting point of potassium formate which is 167.5 °C, more preferably at least at 180 °C. The process is carried out at a temperature of less than 260°C, preferably less than 240 °C, more preferably less than 220°C, more preferably of at most 210 °C, more preferably at less than 210 °C. The pressure can vary widely and can be both above and below ambient pressure. For ease of operation, the process preferably is carried out at ambient pressure or below ambient pressure. The process can be carried out under inert atmosphere. The process preferably is carried out in the presence of hydrogen, more preferably the gas present consists for at least 90 % of hydrogen at the start of the process, more preferably the gas consists for at least 95 % of hydrogen. Most preferably, the process is carried out under hydrogen atmosphere. As formate coupling generates hydrogen, a hydrogen atmosphere allows to use further use the hydrogen gas separated from the product without additional purification.

Preferably, the mixture containing base catalyst and potassium formate is predominantly free of oxygen, moisture and other protic components at the start of the process. In order to ensure that the metal hydride is not converted into an inactive compound, it is preferred that the potassium formate contains less than 0.5 mole % of total amount of water and other protic compounds, based on molar amount of potassium formate. Preferably, the amount of water and other protic compounds is at most 0.2 mole %, more preferably at most 0.1 mole %, more preferably at most 0.05 mole %, based on molar amount of potassium formate. Most preferably, the reaction mixture is free from water. Water present in the reaction mixture will reduce the effective amount of base catalyst.

The current process may also include other additives such as inorganic or organic compounds. It is possible to include one or more additives which reduce the potassium formate melting point and thereby reduce the reaction temperature. This may improve the yield of the process. In addition, the reaction mixture may contain organic solvent, such as an ether. The solvents added preferably are recovered after the reaction.

The reaction mixture preferably is a liquid which is substantially free from water and other protic compounds. The reaction mixture preferably comprises potassium formate, base catalyst and at most 10 % by weight of a further aprotic compound. The amount of further aprotic compound preferably is at most 5 %, more preferably at most 2 % by weight.

The present process was found to convert the potassium formate to oxalate in high yield. It was found to be possible to achieve a yield of at least 80 % of the theoretical maximum yield of oxalate, more specifically at least 85 %, more specifically at least 90 % and most specifically at least 95 %.

It is to be understood that the following examples are exemplary and explanatory only and are not necessarily restrictive of the present disclosure.

### EXAMPLES

Potassium formate was dried overnight in a vacuum oven at 110 °C and transferred to a glove box. Hydride catalysts were transferred to the glove box directly. Reaction mixtures for catalyst screening were prepared in batches of different weight percent ratios of catalyst to formate. For each batch, 3-5 g of dry potassium formate or potassium oxalate (for product stability testing) were crushed in a ceramic mortar until a fine powder was obtained. After 5 minutes of mixing the batch was transferred to a glass vial with a plastic septum cap. Various amounts of catalyst were weighed and mixed with the potassium formate. Standard batch size was 5 g to reduce the weighing error. For each reaction, 290-310 mg of the formate and catalyst mixture was used. The exact mass was measured and used to calculate theoretical yields of produced gases and solids.

The reaction onset and the formate conversion were studied by time-resolved volumetric measurements of the gas produced during the reaction. This was performed with a digital kinetic evaluation device called a Bubble-Counter described by T.K. Slot et al. in Angew. Chemie - Int. Ed. 2019, 58, 17273-17276. This device is capable of measuring gas production over time (so-called "bubble counting") using an internal proportional integral dervative (PID) loop. Each gas bubble is recorded as a string of values. The gases are subsequently qualitatively analyzed using mass spectroscopy or gas chromatography. The string includes the time when the bubble was counted and its size. Calibration of the system allowed the conversion of gas volume produced to reaction progress. This way the reaction can be studied in a broad composition and temperature range with high time resolution. For all experiments, 7 mL glass vials were used as reactors in a ring-shaped heating element connected to the PID controller in the Bubble-Counter device.

For the hydride catalysts the oxalate yields were determined at 200 °C isothermal reaction temperature. At this temperature all reactions according to the invention were complete within 2 minutes (visible by the termination of the hydrogen evolution). For non-isothermal measurements, to identify the reaction onset temperature, the reaction vial was ramped to a setpoint temperature with a ramp rate of 2 °C min⁻¹. For reactions at lower temperatures, the reaction vessel was heated from room temperature to a setpoint temperature of 210 °C. For higher temperature reactions using KOH as catalyst, or for the reactions without catalyst the reaction was ramped from room temperatures to 420 °C with 2°C/min. The reactions with KOH as a catalyst and without catalyst were typically complete in 10-20 minutes (visible by the termination of the hydrogen evolution). After holding the sample for 20 min at the target temperature, the reactor was taken out of the system and cooled to room temperature. The remaining catalyst was then quenched with 1 mL of distilled water.

A Tornado Hyperflux Pro Raman spectrometer equipped with an Axiom RFP 400 probe and a laser with a 785 nm wavelength was used for in-situ analysis of the reaction mixture. A hole in the bottom of the heating element was used to access the reactor from the bottom during the reaction. A time resolution of 900 ms was realized. During the reaction, simultaneously also the volume and composition of the gases were analyzed online. We used this system to follow the reaction and to further confirm the reaction progression by an additional means and to attempt identifying intermediates.

After the reaction, products were characterized using the different absorbance bands of oxalate, formate, and carbonate in Fourier Transform Infra Red (FTIR) spectroscopy. The produced gases were analysed by mass spectrometry and gas chromatography. ¹H NMR and ¹³C NMR spectroscopy were used to confirm the molecular structures of products and to investigate the presence of intermediates. Isotope labeling combined with mass spectrometry was used to identify possible carbonite intermediate. Raman spectroscopy was used to identify possible reaction mechanism and to follow the reaction time-resolved spectroscopically. We used a SmartSeal liquid cell from PIKE technologies with CaF₂ windows and cavity thickness of 0.025 mm. The spectra were recorded with a Varian 660-IR spectrometer. The cell was flushed with nitrogen gas for 5 - 10 min before measurement. After scanning the background of the cell, the water sample was scanned as a reference. Each sample was scanned thrice in the 400 - 4000 cm⁻¹ range to ensure the reproducibility. After each measurement, the cell was flushed with deionized water to remove traces of the previous sample. The sample volume for the infrared measurement was around 0.5 mL. All experiments were performed at least three times and all results in Table 1 are the average of these replicates. In all cases the reproducibility was very good.

**Table 1**

| **Catalyst** | **Catalyst Loading (wt. %)** | **Temperature range (onset temp) (°C)** | **Oxalate yield for isothermal reaction at 200°C** |
|---|---|---|---|
| **NaH** | 0.1 | 157 - 162 | 22% |
| | 0.5 | 157 - 162 | 99% |
| | 1.0 | 157 - 162 | 97% |
| | 2.5 | 157 - 162 | 94% |
| | 5.0 | 156 - 161 | 98% |
| | 10.0 | 156 - 158 | 97% |
| **KH** | 0.1 | 158 - 163 | 69% |
| | 0.5 | 155 - 161 | 98% |
| | 1.0 | 155 - 160 | 94% |
| | 2.5 | 152 - 155 | 98% |
| | 5.0 | 157 - 162 | 99% |
| | 10.0 | 153 - 156 | 98% |
| **LiH** | 1.0 | 161 - 163 | 84% |
| | 5.0 | 152 - 154 | 96% |
| | 10.0 | 153 - 158 | 98% |

| **Comparative examples** | | | |
|---|---|---|---|
| **KOH** | 5 | 318 - 348 | 83% ^{(at 420°C)} |
| **No catalyst** | 0 | 363 - 409 | 21% ^{(at 420°C)} |

## Claims

1. A process for preparing potassium oxalate which process comprises contacting potassium formate with a base catalyst at a temperature of from 140 to 260 °C which base catalyst is a metal hydride.

2. The process according to claim 1 in which process the reaction mixture is free from water.

3. The process according to claim 1 or 2 in which process the temperature is of from 150 to at most 220 °C.

4. The process according to any one of claims 1 to 3 wherein the potassium formate is prepared by converting potassium carbonate and/or potassium bicarbonate to potassium formate.

5. The process according to any one of claims 1 to 4 wherein the base catalyst is an alkali metal hydride.

6. The process according to any one of claims 1 to 5 wherein the amount of base catalyst is at most 2.5 % by weight based on total amount of potassium formate.

7. The process according to any one of claims 1 to 6 wherein the amount of base catalyst is at least 0.01 % by weight.

8. The process according to any one of claims 1 to 7 wherein further is present an additive reducing the potassium formate melting point.

9. A process for preparing oxalic acid which process comprises (i) preparing potassium oxalate in a process according to any one of claims 1 to 8, and (ii) converting the potassium oxalate into oxalic acid.

## Patentansprüche

1. Verfahren zum Herstellen von Kaliumoxalat, wobei das Verfahren ein Inkontaktbringen von Kaliumformiat mit einem basischen Katalysator bei einer Temperatur von 140 bis 260 °C umfasst, wobei der basische Katalysator ein Metallhydrid ist.

2. Verfahren nach Anspruch 1, wobei in dem Verfahren das Reaktionsgemisch wasserfrei ist.

3. Verfahren nach Anspruch 1 oder 2, wobei in dem Verfahren die Temperatur von 150 bis höchstens 220 °C beträgt.

4. Verfahren nach einem der Ansprüche 1 bis 3, wobei das Kaliumformiat durch Umwandeln von Kaliumcarbonat und/oder Kaliumbicarbonat in Kaliumformiat hergestellt wird.

5. Verfahren nach einem der Ansprüche 1 bis 4, wobei der basische Katalysator ein Alkalimetallhydrid ist.

6. Verfahren nach einem der Ansprüche 1 bis 5, wobei die Menge des basischen Katalysators höchstens 2,5 Gew.-%, basierend auf einer Gesamtmenge an Kaliumformiat, beträgt.

7. Verfahren nach einem der Ansprüche 1 bis 6, wobei die Menge des basischen Katalysators mindestens 0,01 Gew.-% beträgt.

8. Verfahren nach einem der Ansprüche 1 bis 7, wobei ferner ein Additiv vorhanden ist, das den Schmelzpunkt von Kaliumformiat senkt.

9. Verfahren zum Herstellen von Oxalsäure, wobei das Verfahren (i) das Herstellen von Kaliumoxalat in einem Verfahren nach einem der Ansprüche 1 bis 8 und (ii) das Umwandeln des Kaliumoxalats in Oxalsäure umfasst.

## Revendications

1. Procédé de préparation d'oxalate de potassium, lequel procédé comprend la mise en contact de formiate de potassium avec un catalyseur de base à une température allant de 140 à 260 °C, lequel catalyseur de base est un hydrure métallique.

2. Procédé selon la revendication 1, dans lequel le mélange réactionnel est exempt d'eau.

3. Procédé selon la revendication 1 ou 2, dans lequel la température va de 150 à au plus 220 °C.

4. Procédé selon l'une quelconque des revendications 1 à 3, dans lequel le formiate de potassium est préparé en convertissant du carbonate de potassium et/ou du bicarbonate de potassium en formiate de potassium.

5. Procédé selon l'une quelconque des revendications 1 à 4, dans lequel le catalyseur de base est un hydrure de métal alcalin.

6. Procédé selon l'une quelconque des revendications 1 à 5, dans lequel la quantité de catalyseur de base est d'au plus 2,5 % en poids sur la base de la quantité totale de formiate de potassium.

7. Procédé selon l'une quelconque des revendications 1 à 6, dans lequel la quantité de catalyseur de base est d'au moins 0,01 % en poids.

8. Procédé selon l'une quelconque des revendications 1 à 7, dans lequel un additif réduisant le point de fusion du formiate de potassium est également présent.

9. Procédé de préparation d'acide oxalique, lequel procédé comprend (i) la préparation d'oxalate de potassium dans un procédé selon l'une quelconque des revendications 1 à 8, et (ii) la conversion de l'oxalate de potassium en acide oxalique.
